## Europäisches Patentamt
## European Patent Office
## Office européen des brevets

(11) Publication number: **0 047 176**
**B1**

(12) **EUROPEAN PATENT SPECIFICATION**

(45) Date of publication of patent specification: **24.04.85**

(51) Int. Cl.⁴: **A 61 B 5/14**

(21) Application number: **81304003.7**

(22) Date of filing: **02.09.81**

(54) Device suitable for sampling blood.

(30) Priority: **02.09.80 US 182874**

(43) Date of publication of application:
**10.03.82 Bulletin 82/10**

(45) Publication of the grant of the patent:
**24.04.85 Bulletin 85/17**

(84) Designated Contracting States:
**AT BE CH DE FR GB IT LI LU NL SE**

(56) References cited:
**WO-A-80/02106**
**US-A-3 865 548**
**US-A-3 943 917**
**US-A-3 955 423**
**US-A-3 960 139**
**US-A-3 978 846**

(73) Proprietor: **WARNER-LAMBERT COMPANY**
**201 Tabor Road**
**Morris Plains New Jersey 07950 (US)**

(72) Inventor: **Ford, George William, Jr.**
**2043 Falcon Hill Drive Sandy**
**Utah 84080 (US)**

(74) Representative: **Jones, Michael Raymond et al**
**HASELTINE LAKE & CO. Hazlitt House 28**
**Southampton Buildings Chancery Lane**
**London WC2A 1AT (GB)**

Courier Press, Leamington Spa, England.

## Description

This invention relates to a device suitable for sampling blood; the device of the present invention is suitable for obtaining arterial blood samples for blood gas analysis.

Measurements of the blood level of carbon dioxide ($P_{CO_2}$) and of oxygen ($P_{O_2}$) as well as blood pH constitute a quick reliable diagnostic regimen for determination of respiratory parameters and acid-base balance. However, as arterial blood rather than venous blood, which is used in most clinical laboratory procedures, must be obtained for such blood gas analysis and as the gases being determined are extremely sensitive to influences from the outside environment, special precautions must be used to preserve the integrity of the sample.

A device which is to be used for sampling arterial blood should function in the following manner. The device should be constructed to fill under arterial blood pressure but should be resistant enough to prevent the lower venous pressures from filling the device. This function allows for discrimination of the sample being obtained. The device should present a barrier to the outside environment which will prevent gas exchange with the ambient atmosphere. The device should have some means of removing from within the device gas trapped within the device, which would allow for gas exchange. Also the device should have fitment means by which a hollow hypodermic needle may be attached to collect the sample. This fitment should be of proper calibre and configuration to allow interface with instrumentation used in blood gas analysis and other purposes.

In U.S. Patent No. 3865548, a syringe device suitable for sampling blood is disclosed which comprises a hollow barrel open at its proximal end and with provision for a needle, a plug assembly comprising a hollow piston within the open end of the barrel for slidable reciprocating movement therein and a membrane element which is pervious to the passage of air therethrough but impervious to the passage of liquid after being wetted.

A predetermined quantity of sample, e.g. expired air or blood, is drawn into sample region. Gases in the sample of liquid are free to pass through the membrane into the test reagent for analytical purposes.

The present invention provides a sampling device having certain similarities to that disclosed above, but having a novel, efficient arrangement for securing the membrane and also a plunger which is open at its proximal end to enable gas to pass from the sample chamber and through the membrane, as blood flows into the sample chamber.

According to the present invention, there is provided a syringe device (10) suitable for sampling blood, the device comprising a hollow barrel (11) open at its proximal end and with provision for a hypodermic needle to be removably mounted at its distal end, and a plunger assembly within the open end of the barrel (11) for slidable reciprocating movement therein, the plunger assembly comprising:—

a hollow piston (12) provided internally with a circular lip (25) formed in the distal end region of the hollow piston (12), the circular lip being of lesser diameter than the hollow piston (12);

an annular fitting (20) which includes a flange (22) having a cylindrical section extending in the proximal direction, the cylindrical section having an internal diameter to cooperate with and receive the circular lip (25) for a force fit therewith and having an external diameter for a force fit within the hollow piston (12), the annular fitting (20) also including an inner shoulder and an outer shoulder, the outer shoulder being adapted to receive a sealing ring (23) for sealing the space between the hollow barrel (11) and hollow piston (12) when the plunger assembly is assembled and the annular fitting (20) is located within the hollow barrel (11), and the inner shoulder cooperating with the circular lip (25) of the hollow piston (12) for supporting and fixedly maintaining a membrane element (21) therebetween;

The sealing ring (23) for reception in said space; and

the membrane element (21), which is maintained between the inner shoulder and circular lip (25) and is pervious to the passage of air therethrough but impervious to the passage of liquid after being wetted.

In the immediately preceding paragraph the presence of reference numerals is intended merely as an aid to the reader, to indicate the specific components illustrated in the drawing which corresponds to the terms associated with the reference numerals, but the presence of the reference numerals is not to restrict in any way the scope of such terms.

For a better understanding of the present invention and to show how the same may be carried into effect, reference will now be made, by way of example, to the accompanying drawing, in which:—

Figure 1 is an exploded view in perspective of an embodiment of the blood sampling device of the present invention, showing the manner in which the several parts are assembled; and

Figure 2 is a cross-sectional view of the blood sampling device in Figure 1 with the several parts in position prior to drawing off an arterial blood sample. Like reference numerals indicate like parts throughout the different views of the drawing.

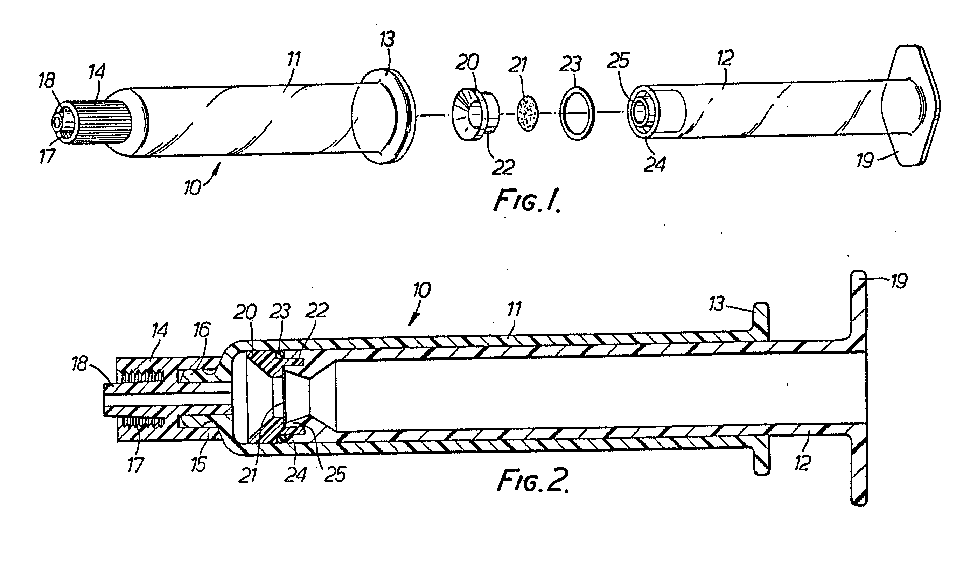

Referring now to both Fig. 1 and Fig. 2 of the drawing, the illustrated embodiment of the blood sampling device of the present invention, generally indicated by the reference numeral 10, comprises a barrel 11 and a plunger assembly whose primary component is a hollow piston 12. Barrel 11 is provided at one end region with a circumferential annular lip 13 and at the other end region with a fitting 14. Barrel 11 may be formed out of glass or of a clear moulded plastics material of a composition such as to present a barrier to atmospheric gas.

When the barrel 11 is moulded, the element forming the fitting 14 may be moulded integrally with the barrel. However, in the embodiment shown, fitting 14 comprises a separate element, as can be seen in Fig. 2. To assemble the parts, the fitting 14 is mounted on the barrel 11 by means of a sleeve or collar portion 15 which fits over a hub 16 moulded as a part of the distal end region of barrel 11, with a suitable adhesive being employed to join the parts or by providing dimensions for a force or friction fit. The interior of the fitting 14 is provided with threads 17 which are shaped to accept a removable hollow hypodermic needle (not shown) whose external hub is provided with complementary lugs which allow for a locking screw assembly or whose inner bore can be mounted by a force fit over the outer surface of a projecting stem 18.

The plunger assembly, which in normal operating position fits into the interior of barrel 11, as shown in Fig. 2, comprises in addition to the hollow piston 12 whose proximal end is shaped to provide a handle 19, a membrane and sealing ring assembly mounted on the distal end region of the piston 12 whose several elements are more particularly shown in exploded view in Fig. 1. The membrane and sealing ring assembly comprises an annular fitting 20, an interior region of which accommodates a membrane medium in the form of a disc 21; the annular fitting 20 has a flange 22 of cylindrical section extending in the proximal direction, on which is seated an O-ring 23. When the several parts are brought together in assembled position, as shown in Figure 2, the O-ring 23 is held in seated position between the annular fitting 20 and an end surface 24 of the piston 12, with the inner surface of the flange 22 of the annular fitting 20 fitting over a circular lip 25 moulded into and forming the end of the piston 12.

Thus, when the parts are assembled, the membrane disc 21 is held firmly in position within the annular fitting 20 by the pressure exerted by the circular lip 25 against the disc 21 when the flange 22 of the annular fitting 20 is placed over the circular lip 25 and firmly seated in position. With the parts as assembled, the O-ring 23 also acts as a seal between the inner surface of the barrel 11 and the outer surface of the piston 12. The structure of membrane disc 21 allows for free passage of gases but this material is structured such that when a liquid such as blood, for example, wets the surface an ionic equilibrium between the surface tension of the liquid and the ionic charge of the membrane is reached and no fluid will pass through the membrane. Suitable materials for this purpose are those known in the art as hydrophobic membranes. Furthermore, when the membrane is in ionic equilibrium with the liquid contained inside the barrel 11, the only means by which gas may now exchange is by Brownian motion across a high resistance. Driving pressures which would allow for such exchanges do not exist in the environment in the barrel 11.

Before final assembly of the piston 12 into the barrel 11, crystalline heparin may be introduced into barrel 11. The presence of heparin acts as an anticoagulant to prevent the blood sample from clotting, which would destroy its integrity for accurate blood gas analysis. As the anticoagulant is in crystalline form, no dilution of the specimen obtained takes place as is the case with heparin in solution. After adding the anticoagulant the plunger assembly 12 is inserted into the barrel assembly 11.

When drawing an arterial blood sample using the blood sampling device illustrated in the drawing, the barrel 11 is held firmly in one hand and the handle 19 is used to retract the piston 12 to a position where the volume of open space in the barrel 11 is equivalent to the volume of the blood sample to be drawn. A hollow hypodermic needle of the usual type (not shown) is then locked under the threads 17 or placed firmly over the stem 18 and the bevelled end of the needle then introduced into an available artery such as the radial or brachial artery. On penetration of the arterial wall, the internal arterial blood pressure will force blood to flow through the needle and into the blood sampling device. As the structure of the membrane disc 21 permits gas to flow through it, while the chamber within the blood sampling device fills with arterial blood the gas present in the barrel 11 will be displaced through the disc 21 and will leave the barrel 11 through the interior of the hollow piston 12. When the blood level reaches the disc 21, an ionic equilibrium between the surface tension of the liquid and the ionic charge of the membrane is reached and a seal is formed preventing the passage of blood through the membrane. The needle is then withdrawn from the artery and the blood sampling device is rotated to dissolve the crystalline anticoagulant into the sample to prevent clotting. Pressure is applied to the arterial wall to allow the normal blood coagulation process to close off the narrow puncture wound. The chamber of the blood sampling device is now sealed by membrane disc 21, and the blood sample may be introduced directly into appropriate analytical instrumentation, which is facilitated by the particular calibre and configuration of the fitting 14.

**Claim**

1. A syringe device suitable for sampling blood, the device comprising a hollow barrel open at its proximal end and with provision for a hypodermic needle to be removably mounted at its distal end, and a plunger assembly within the open end of the barrel for slidable reciprocating movement therein, the plunger assembly comprising:—

a hollow piston provided internally with a circular lip formed in the distal end region of the hollow piston, the circular lip being of lesser diameter than the hollow piston;

an annular fitting which includes a flange having a cylindrical section extending in the proximal direction, the cylindrical section having an internal diameter to cooperate with and receive the circular lip for a force fit therewith and having an external diameter for a force fit within the hollow piston, the annular fitting also including an inner shoulder and an outer shoulder, the outer shoulder being adapted to receive a sealing ring for sealing the space between the hollow barrel and hollow piston when the plunger assembly is assembled and the annular fitting is located within the hollow barrel, and the inner shoulder cooperating with the circular lip of the hollow piston for supporting and fixedly maintaining a membrane element therebetween;

the sealing ring for reception in said space; and

the membrane element, which is maintained between the inner shoulder and circular lip and is pervious to the passage of air therethrough but impervious to the passage of liquid after being wetted.

## Patentansprüch

1. Spritzenvorrichtung für Blutproben, mit einem hohlen an seinem proximalen Ende offenen und an seinem distalen Ende zur entfernbaren Anbringung einer subkutanen Spritzennadel ausgelegten Zylinder und einer Plungeranordnung innerhalb des offenen Endes des Zylinders zum gleitenden Oszillieren innerhalb desselben, welche Plungeranordnung folgendes umfaßt:

einen hohlen Kolben, der innen mit einer im distalen Endbereich des hohlen Kolbens ausgebildeten kreisförmigen Lippe von geringerem Durchmesser als der hohle Kolben versehen ist;

ein ringförmiges Anschlußstück, enthaltend einen Flansch mit einem sich in proximale Richtung erstrekkenden zylindrischen Abschnitt, welcher zylindrische Abschnitt einen Innendurchmesser zum Zusammenwirken mit der kreisförmigen Lippe bzw. zur Aufnahme derselben zwecks Preßpassung mit derselben und einen Außendurchmesser zur Preßpassung innerhalb des hohlen Kolbens aufweist, wobei das ringförmige Anschlußstück auch eine Innenschulter und eine Außenschulter aufweist, welche Außenschulter zur Aufnahme eines Dichtungsrings zum Dichten des Raums zwischen dem hohlen Zylinder und dem hohlen Kolben, wenn die Plungeranordnung zusammengesetzt ist und sich das ringförmige Anschlußstück innerhalb des hohlen Zylinders befindet, ausgelegt ist und welche Innenschulter mit der kreisförmigen Lippe des hohlen Kolbens zwecks Haltens und Fixierens eines Membranelements zwischen ihnen zusammenwirkt;

den Dichtungsring zur Aufnahme in diesem Raum; und

das Membranelement, das zwischen der Innenschulter und der kreisförmigen Lippe gehalten und durchlässig für einen Luftdurchfluß, aber nach Anfeuchten undurchlässig für einen Flüssigkeitsdurchfluß ist.

## Revendication

1. Dispositif à seringue utilisable pour l'échantillonnage de sang, ce dispositif comprenant un cylindre creux ouvert à son extrémité proximale et muni de moyens de montage amovible d'une aiguille hypodermique à son extrémité distale, ainsi qu'un ensemble plongeur monté dans l'extrémité ouverte du cylindre de manière à pouvoir effectuer un mouvement de coulissement en va et vient dans celui-ci, cet ensemble plongeur comprenant:

un piston creux muni intérieurement d'une lèvre circulaire ménagée dans la zone de l'extrémité distale du piston creux, la lèvre circulaire ayant un diamètre inférieur à celui du piston creux;

une garniture annulaire comprenant un rebord à section cylindrique et s'étendant dans la direction proximale, le diamètre intérieur de la section cylindrique étant tel que celle-ci coopère avec la lèvre circulaire et la recoive avec un ajustement à force, et le diamètre extérieur de la section cylindrique étant tel que celle-ci soit reçu dans le piston creux avec un ajustement à force, la garniture annulaire comprenant également un épaulement intérieur et un épaulement extérieur, l'épaulement extérieur étant appelé à recevoir une bague d'étanchéité pour obturer l'espace entre le cylindre creux et le piston creux lorsque l'ensemble plongeur est monté et la garniture annulaire disposée dans le cylindre creux, l'épaulement intérieur coopérant avec la lèvre circulaire du piston en creux pour supporter et maintenir fixe un élément à membrane entre eux;

la bague d'étanchéité étant appelée à être dans ledit espace; et

la membrane étant maintenue entre l'épaulement intérieur et la lèvre circulaire et perméable à l'air mais imperméable au liquide après avoir été humectée.

Fig. 1.

Fig. 2.